(19) **Europäisches Patentamt / European Patent Office / Office européen des brevets**

(11) **EP 4 142 678 B1**

(12) **FASCICULE DE BREVET EUROPEEN**

(45) Date de publication et mention de la délivrance du brevet:
**29.05.2024 Bulletin 2024/22**

(21) Numéro de dépôt: **21732409.4**

(22) Date de dépôt: **30.04.2021**

(51) Classification Internationale des Brevets (IPC):
**A61K 8/06** *(2006.01)*  **A61K 8/31** *(2006.01)*
**A61K 8/92** *(2006.01)*  **A61Q 13/00** *(2006.01)*

(52) Classification Coopérative des Brevets (CPC):
**A61K 8/06; A61K 8/31; A61K 8/922; A61Q 13/00;**
A61K 2800/30

(86) Numéro de dépôt international:
**PCT/FR2021/050749**

(87) Numéro de publication internationale:
**WO 2021/219968 (04.11.2021 Gazette 2021/44)**

(54) **COMPOSITION PARFUMANTE**

DUFTSTOFFZUSAMMENSETZUNG

FRAGRANT COMPOSITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2020 FR 2004314**

(43) Date de publication de la demande:
**08.03.2023 Bulletin 2023/10**

(73) Titulaire: **LVMH Recherche**
**45800 Saint Jean de Braye (FR)**

(72) Inventeurs:
- **CHAMPREDONDE, Elodie**
  **45800 SAINT JEAN DE BRAYE (FR)**
- **BOUCHARD DE LA POTERIE, Valérie**
  **45800 SAINT JEAN DE BRAYE (FR)**

(74) Mandataire: **Cabinet Beau de Loménie**
**158, rue de l'Université**
**75340 Paris Cedex 07 (FR)**

(56) Documents cités:
**WO-A2-2014/155019     FR-A1- 2 952 531**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

Domaine Technique

La présente invention se rapporte à une nouvelle composition cosmétique parfumante qui peut être une eau de toilette, une eau de Cologne, un extrait de parfum, un esprit de parfum ou une eau de parfum selon la teneur en concentré de parfum qu'elle contient et les notes qu'elle exprime. Cette composition parfumante a d'excellentes propriétés telles que la fidélité olfactive du concentré de parfum et la rémanence de la note parfumée au cours du temps après application, l'absence de sensation de collant au toucher, et l'aptitude à la pulvérisation.

Pour fabriquer des compositions parfumantes qui contiennent de l'eau, la dispersion du concentré de parfum dans de l'eau n'est pas possible car le concentré s'hydrolyse et développe une odeur rédhibitoire: les fonctions esters des molécules présentes dans le concentré s'hydrolysent en présence d'eau pour former des acides qui développent une odeur acétique caractéristique de ces fonctions. C'est pourquoi, pour préparer des compositions parfumantes hydroalcooliques, un concentré de parfum est solubilisé dans un alcool tel que l'éthanol, avant sa dispersion dans l'eau.

Or, l'utilisation d'alcool est remise en cause en raison de risques d'intolérance, d'allergie, d'irritation, de photosensibilisation chez certains utilisateurs, et de risques de sécurité liés à l'utilisation d'alcool. Par ailleurs, certaines coutumes religieuses interdisent son usage.

Une première façon de préparer une composition parfumante sans alcool est d'utiliser d'autres composés pour disperser le concentré de parfum dans l'eau, par exemple un agent solubilisant et un agent émulsionnant. Cependant, les solubilisants et les émulsionnants ont une influence négative sur les propriétés olfactives et organoleptiques du produit : la note olfactive peut être altérée dans le temps et le toucher de la composition peut donner une sensation de collant. Afin d'éviter ces inconvénients, les solubilisants et les émulsionnants de concentré parfumant doivent être utilisés en faibles quantités, ce qui limite le taux de concentré de parfum que l'on peut introduire dans une composition parfumante. C'est le cas des compositions sans alcool décrites dans la demande FR 2 952 531 qui contiennent un agent solubilisant, un agent émulsionnant et des faibles quantités de concentré de parfum, typiquement de l'ordre de 2%.

Une deuxième façon de supprimer l'alcool dans une composition parfumante tout en préservant la stabilité du concentré est de supprimer l'eau. Les compositions parfumantes sans alcool anhydres sont des compositions huileuses, des sticks, des poudres ou des concrètes. Ces produits présentent des textures solides ou liquides visqueuses, qui sont très différentes des compositions alcooliques ou hydroalcooliques classiques. Ces compositions ne sont pas pulvérisables, peuvent tacher les vêtements, et ne peuvent être utilisées qu'en application directe sur la peau, ce qui constitue une vraie limitation d'usage. Elles nécessitent par conséquent d'adapter le packaging et la gestuelle d'application, ce qui n'est pas toujours accepté par le consommateur. En outre, ces compositions sont perçues comme grasses et n'apportent aucune fraîcheur.

Au regard de tous les inconvénients listés précédemment, le besoin subsiste de proposer une composition parfumante aqueuse contenant des teneurs élevées en concentré de parfum et des teneurs réduites, voire nulles, en éthanol, et qui présente des propriétés organoleptiques supérieures ou égales à celles des compositions de l'art antérieur.

Le besoin subsiste notamment de proposer une composition parfumante contenant des teneurs élevées en concentré de parfum qui présente une bonne intensité de diffusion et/ou une bonne rémanence, qui est pulvérisable, dont l'identité olfactive est stable dans le temps sans dégradation du concentré parfumant, et/ou dont le toucher est non gras et non collant. L'invention vise à atteindre au moins un des objectifs précités.

Description générale de l'invention

L'invention propose donc une cosmétique parfumante liquide comprenant a) au moins 50% en masse d'eau, b) moins de 10% en masse d'éthanol, c) au moins 3% en masse d'un concentré de parfum , d) au moins une huile choisie parmi les alcanes, et e) moins de 0;1% en masse d'un agent émulsionnant, les pourcentages étant exprimés par rapport à la masse de la composition.

Brève description des dessins

[Fig. 1] La Figure 1 présente l'évolution de l'intensité olfactive (MD BB) d'une composition de l'invention et d'un témoin dans le temps à T0, T1 et T2.

[Fig. 2] La Figure 2 présente l'évolution de la fidélité olfactive (MD BB) d'une composition de l'invention et d'un témoin dans le temps à T0, T1 et T2. Description des modes de réalisation

On entend par « composition parfumante » ou « parfum », un produit sous forme liquide destiné à parfumer un individu après sa pulvérisation ou son application sur la peau, les cheveux ou les vêtements. Un tel produit n'est pas rincé après application. On distinguera ainsi une composition parfumante d'une composition parfumée. Une composition cosmétique peut être parfumée sans être parfumante.

Dans le cadre de l'invention, l'expression « de...à » entend inclure les bornes de la plage de valeurs, à la différence de l'expression « entre...et » qui entend exclure les bornes de la plage de valeurs. De préférence, la composition selon l'invention présente deux phases visuellement distinctes au repos.

Elle peut se présenter sous forme de biphase comprenant deux phases visuellement distinctes non miscibles au repos (de préférence une phase aqueuse et une phase huileuse), qui se mélangent facilement par agitation pour permettre une application homogène, et qui déphasent rapidement après arrêt de l'agitation pour retrouver leur état initial. De préférence, la composition présente deux phases visuellement distinctes superposées, présentant de préférence une interface nette. De préférence encore, l'une des phases, de préférence les deux phases, sont transparentes.

La composition contient un concentré de parfum, encore dénommé « concentré parfumant ». Le concentré de parfum peut être par exemple choisi parmi des composés dont le nom INCI figure sur la liste des ingrédients d'une composition parfumante proposée à la vente sous le nom de « Parfum ». Un concentré de parfum est un composé ou un mélange de composés au moins partiellement volatils à température ambiante, dont on détecte l'odeur. Un concentré de parfum constitué d'huiles essentielles doit nécessairement être dilué pour exprimer tout son potentiel olfactif, à savoir une perception évoluant au cours de la journée après application sur la peau, dictée par la présence de composés organiques odorants ayant des volatilités très différentes les uns des autres. L'élaboration d'un concentré de parfum consiste à associer plusieurs matières parfumantes pour donner à la composition parfumante une note de têtes, une note de coeur et note de fond. Un concentré de parfum utilisé dans le cadre de l'invention comprend de préférence majoritairement des notes de têtes et des notes de coeur.

Le concentré de parfum est préparé à partir de matières parfumantes organiques naturelles ou synthétiques.

Comme matières parfumantes d'origine naturelle, on peut citer par exemple les extraits de fleurs, de tiges et de feuilles, de fruits, d'écorces de fruits, de racines, de bois, d'herbes et de graminées, de résines et de baumes. Ces matières parfumantes végétales peuvent être des huiles essentielles, comme par exemple les essences de bergamote, de rose, de lavande, de santal, de cardamone, de sauge, de camomille, de girofle, de mélisse, de menthe, de feuilles de cannelier, de genièvre, de vétiver, d'oliban, de galbanum et de labdanum.

Comme matières parfumantes d'origine synthétique, on peut citer par exemple l'hédione, l'éthylène brassilate et l'habanolide, l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle, le benzyléthyléther, les alcanals linéaires comportant de 8 à 18 atomes de carbone, le citral, le citronellal, le citronellyloxyacétaldéhyde, le cyclamènaldéhyde, l'hydroxycitronellal, les ionones comme l'alpha-isométhylionone, et la méthylcédrylcétone, l'anéthol, le citronellol, l'eugénol, l'isoeugénol, le géraniol, le linalol, le phényléthylalcool, le terpinéol, les terpènes. Ces composés se présentent souvent sous forme de mélange de deux ou plus de ces substances odorantes.

On classe souvent les notes olfactives dans des familles qui permettent à un consommateur de savoir à quelle perception il doit s'attendre. On peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères, les cuirs et leurs mélanges.

Le concentré de parfum représente au moins 3% en masse, de préférence au moins 4% en masse, par exemple au moins 5% en masse, de préférence encore au moins 6%, 7% ou 8% en masse, mieux au moins 10%, 11 %, 12%, 13% ou 14% en masse, et mieux encore au moins 15% en masse de la masse de la composition.

La concentration du concentré de parfum peut aller de 3% en masse à 40% en masse ou être comprise entre 3% et 40% en masse. Elle va, de préférence de 4% à 38% en masse ou de 5% en masse à 35% en masse, par exemple de 7% en masse à 30% en masse ou de 10% en masse à 25% en masse de la masse de la composition. Dans un mode de réalisation, le concentré de parfum représente de 5% en masse à 15% en masse de la masse de la composition.

La teneur en éthanol dans la composition est inférieure à 10% en masse par rapport à la masse de la composition, de préférence inférieure à 5%, et de façon encore plus préférée inférieure à 2%. La composition est de préférence exempte d'éthanol, ajouté au cours de la préparation de la composition parfumante par mélange des différents ingrédients. On ne peut exclure qu'il existe des traces d'éthanol dans certains des ingrédients utilisés.

La composition de l'invention contient moins de 0,5%, de préférence moins de 0,1% en masse d'un agent émulsionnant tel qu'un agent tensioactif ou un polymère émulsionnant, qui risquent de dégrader les propriétés olfactives et organoleptiques de la composition. Elle contient de préférence moins de 0,05% en masse d'un agent émulsionnant, et de façon encore préférée moins de 0,01% en masse de la composition. Selon un mode particulièrement préféré, la composition en est exempte.

En particulier la composition comprend moins de 0,5% en masse, de préférence moins de 0,1% en masse de un ou plusieurs tensioactifs non ioniques, de tensioactifs polyoxyalkylénés comprenant au moins cinq motifs choisis parmi -CH2CH(OH)CH2- et -OCH2CH2-, tels que les composés polyoxyéthylénés et les composés polyoxypropylénés. On peut citer parmi ces tensio-actifs les éthers polyoxyalkylénés, comme le POE(10) cetyl éther, les esters polyoxyalkylénés, comme le PEG-40 hydrogenated castor oil ou le POE(20) sorbitan monolaurate, les condensats d'alkylphénols polyoxyéthylénés, les produits de condensation d'oxyde d'éthylène avec le produit de réaction d'oxyde de propylène et de l'éthylène diamine, les alcools polyéthoxylés, les polysorbates, et les diméthicone copolyols. La composition est de préférence exempte en tensioactif non ionique.

Par polymère émulsionnant on entend un polymère amphiphile c'est-à-dire doté d'au moins une partie hydrophile et d'au moins une partie hydrophobe. Les groupements hydrophiles et les groupements hydrophobes sont bien connus de l'homme du métier, susceptible d'aider à former et stabiliser des émulsions. On peut citer à titre d'exemples de polymères amphiphiles émulsionnants les polymères et copolymères à base d'AMPS, réticulés ou non réticulés, les copolymères acide polyacrylique/acrylates d'alkyle, en particulier les copolymères acrylate/C1 0-C30-alkylacrylate et leurs mélanges. La composition comprend de préférence moins de 0,5% en masse, de préférence moins de 0,1% en masse de un ou plusieurs polymères émulsionnants.

La composition est de préférence dépourvue de tout agent émulsionnant.

L'huile est choisie parmi les alcanes, de préférence les alcanes linéaires ou ramifiés, en C8-C19, les polyisobutènes hydrogénés, le squalane, l'hemisqualane et leurs mélanges. On entend par alcane un composé constitué de carbone et d'hydrogène.

De préférence, l'huile alcane est volatile. Par « huile volatile », on entend une huile ayant perdu plus de 20% en poids de sa masse à 15 minutes, plus de 40% en poids de sa masse à 30 minutes et plus de 70% en poids de sa masse à 60 minutes, selon le protocole qui suit :

20mg de l'huile à étudier est pesée sur une plaque de PMMA de 5cm x 5cm à l'aide d'une micropipette et d'une balance de précision. Cette matière est étalée au doigt sur l'ensemble de la plaque. Puis, celle-ci est déposée dans une enceinte ventilée thermostatée à 25° C et 50% d'humdité. Pour chaque matière, le test est réalisé 3 fois. La perte en masse au cours du séchage est mesurée après 15 minutes, 30 minutes et 60 minutes. La masse perdue est exprimée selon le calcul suivant :

[Math.1]

$$\% \: masse \: perdue = \frac{m_{t0} - m_{tx}}{m_{t0}} \times 100$$

Avec *mtx* correspondant à la masse restante au temps mesuré (t15min, t30min ou t60min) et mt0 correspondant à la masse appliquée initialement.

Les mesures de volatilité des huiles sont exprimées en temps (en minute). L'homme du métier pourra ainsi définir les huiles qui conviennent à l'invention sur la base de ce test de suivi de sa perte en masse en fonction du temps.

A titre d'huile alcane, on peut citer notamment :

- les alcanes ramifiés en C8-C16 comme les iso-alcanes (appelées aussi isoparaffines) en C8-C16, l'isododécane, l'isodécane, l'isohexadécane, et par exemple les huiles vendues sous les noms commerciaux d'Isopars ou de Permetyls,
- les alcanes linéaires ou ramifiés ayant des chaînes hydrocarbonées en
- C9-C17, C10-C14, tel qu'un mélange de l'undécane et de tridécane, commercialisé par BASF Care Créations sous la dénomination Cetiol® Ultimate,
- C15-C19, tel que ceux commercialisés par Seppic sous la dénomination Emogreen® L15,
- C9-C12, C12-C14, tel que ceux commercialisés par BIOSYNTHIS sous la dénomination Vegelight® Silk (nom INCI C9-12 ALKANE), Vegelight® 1214LC,
- le n-dodécane (C12) et le n-tétradécane (C14), notamment vendus par Sasol respectivement sous les références PARAFOL® 12-97 et PARAFOL® 14-97, et leurs mélanges.

On peut encore citer le squalane, l'hemisqualane d'origine végétale, minérale ou synthétique comme par exemple l'hémisqualane végétal tel que celui commercialisé par la société Amyris sous la référence Neossance® hemisqualane, le squalane végétal commercialisé par exemple sous la dénomination Squalive® par la société Biosynthis , ou encore les hydrocarbures comme le polyisobutène hydrogéné ayant une masse moléculaire inférieure à 250 g/mol, comme celui portant le nom INCI HYDROGENATED POLYISOBUTENE, tels que les produits de noms commerciaux Parleam®, en particulier le PARLEAM®3 ou le produit Polysynlane®4 vendu par la société NOF CORPORATION, et leurs mélanges.

L'huile alcane est avantageusement présente en une quantité suffisante pour solubiliser le concentré de parfum.

L'huile alcane représente de préférence au plus 20% en masse, de préférence au plus 15% en masse, de préférence encore au plus 10% en masse, et de manière particulièrement préférée au plus 5% en masse de la masse de la composition. Elle peut aller de 1% à 10% en masse ou être comprise entre 1% et 10% en masse. Elle va de préférence de 1% à 8% masse ou de 2% à 7% en masse, et encore mieux de 2% à 5% en masse de la masse de la composition. Dans un mode de réalisation, la composition contient de 1% à 5% en masse d'une huile alcane volatile ou d'un mélange d'huiles alcanes volatiles, lorsque la composition en contient plusieurs.

Le ratio massique entre l'huile alcane et le concentré de parfum est avantageusement supérieur à une valeur minimale choisie dans le groupe constitué par 1/20, 1/10, 1/5, et inférieur à une valeur maximale choisie dans le groupe constitué par 3/1, 2/1, 1/1 et 1/2. Dans un mode de réalisation, le ratio massique entre l'huile alcane et le concentré de parfum va de 1/10 à 2/1 ou de 1/5 à 1/1.

La composition selon l'invention comprend au moins 50% en masse d'eau, de préférence au moins 60% en masse, et mieux au moins 65% en masse. La teneur en eau dans la composition peut être ainsi supérieure à une valeur minimale égale à 70%, 75%, 80% ou 85% en masse par rapport à la masse de la composition. La teneur en eau va par exemple de 75% à 95% en masse de la masse de la composition.

On préfère que la somme des pourcentages massiques de l'eau, du concentré de parfum et de l'huile alcane soit supérieure ou égale à 95% en masse, de préférence encore supérieure à 98% en masse de la masse de la composition.

Avantageusement, la composition de l'invention est essentiellement constituée d'eau, du concentré de parfum et de l'huile alcane. Par « essentiellement constituée », on entend que la somme des pourcentages de l'eau, du concentré de parfum et de l'huile alcane est supérieure ou égale à 98% en masse, voire supérieure ou égale à 99% en masse.

Outre les ingrédients décrits précédemment, la composition peut contenir au moins un ingrédient cosmétiquement acceptable choisi parmi les colorants, les conservateurs, les ajusteurs de pH, les électrolytes, les filtres UV, et les antioxydants.

Ces ingrédients additionnels doivent être olfactivement neutres pour ne pas altérer l'odeur de la composition parfumante, ni provoquer un changement de couleurs, l'apparition d'un trouble lié à des problèmes de stabilité.

Les actifs cosmétiques peuvent être par exemple choisis avantageusement parmi ceux ayant une action sur le soin de la peau tels que des actifs anti-âge, des agents hydratants ou tout autre agent cosmétiquement actif dont l'ajout dans la composition de l'invention permet d'obtenir un effet bénéfique pour la peau tout en conservant les propriétés organoleptiques de la composition.

Les colorants sont par exemple: le caramel, Yellow 5, Acid Blue 9/ Blue 1, Green 5, Green 3 / Fast Green FCF 3, Orange 4, Red 4 / Food Red 1, Yellow 6, Acid Red 33 / Food Red 12, Red 40, le carminé de cochenille (CI 15850, CI 75470), Ext. Violet 2, Red 6-7, Ferric Ferrocyanide, Ultramarines, Acide Yellow 3 / Yellow 10, Acid Blue 3, Yellow 10. Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou. Les colorants représentent généralement de 0,01 à 1%, de préférence de 0,05 à 0,5%, en masse de la masse de la composition parfumante.

Parmi les antioxydants et les conservateurs, on peut citer par exemple l'acide ascorbique, le di-tert-butyl-p-hydroxytoluène (encore appelé BHT ou 2,6-di-tert-butyl-p-crésol), le BHA (tert-butyl-4-hydroxyanisole), les tocophérols comme la vitamine E, les dérivés du tocophérol tels que l'acétate de tocophéryle, l'acide gallique et ses dérivés, le sorbate de potassium, le benzoate de sodium, l'hydroxyacétophénone, et la chlrorphénésine.

Selon un mode de réalisation particulier, la composition parfumante contient de 8% à 12% de concentré de parfum, de 1% à 5% d'huile(s) alcane(s), et de l'eau dans une quantité telle que la somme des concentrations en concentré de parfum, en huile(s) alcane(s) et en eau est supérieure à 99%, les pourcentages étant exprimés en masse par rapport à la masse de la composition.

La composition peut éventuellement contenir, outre les ingrédients décrits précédemment, un composé huileux additionnel, un agent épaississant ou une poudre. Dans un mode de réalisation de l'invention, la composition de l'invention ne comprend aucun de ces ingrédients.

La composition contient une phase aqueuse et une phase grasse, la phase grasse contenant une huile alcane et un concentré de parfum. On préfère que la phase grasse contienne moins de 1% en masse, de préférence moins de 0,1% en masse, d'un composé huileux additionnel.

La composition parfumante peut être conditionnée dans un récipient éventuellement doté d'un moyen d'application. Le moyen d'application peut être un moyen de pulvérisation, une bille ou un bouchon.

L'invention a également pour objet un flacon muni d'un moyen d'application et d'un moyen de conditionnement, et contenant la composition décrite précédemment. Le moyen de conditionnement est de préférence transparent afin de laisser percevoir la composition de l'invention, qui est de préférence elle-même transparente. Le moyen d'application peut être une pompe manuelle, une bille ou un bouchon. Les compositions peuvent être appliquées sous forme de fines gouttelettes au moyen de dispositifs de pressurisation. Ces dispositifs sont bien connus de l'homme de l'art et comprennent les pompes non-aérosols ou "atomiseurs", les récipients aérosols comprenant un propulseur ainsi que les pompes aérosols utilisant l'air comprimé comme propulseur.

Les compositions et les flacons de l'invention peuvent être sous la forme d'eau de toilette, d'eau de Cologne, d'extrait de parfum, de voile de parfum, d'esprit de parfum ou d'eau de parfum. Conformément aux connaissances de l'homme du métier, une eau de Cologne contient généralement de 3% à 8% de concentré de parfum, une eau de toilette en contient généralement de 5% à 15%, une eau de parfum en contient généralement de 12% à 20%, un extrait de parfum ou un esprit de parfum en contient généralement plus de 15 %, et un voile de parfum en contient généralement de 5% à 8%, les pourcentages étant exprimés en masse par rapport à la masse de la composition.

L'invention a pour autre objet, un procédé cosmétique comprenant une étape d'application sur les matières kératiniques ou les vêtements d'une personne, d'une composition cosmétique décrite précédemment. Ce procédé peut être un procédé pour parfumer la peau ou les cheveux d'un individu, qui consiste à appliquer, à l'aide d'un moyen de pulvérisation, sur la peau ou les cheveux de l'individu, la composition décrite précédemment. La composition peut également être appliquée sur les vêtements ; on préfère cependant l'appliquer directement sur la peau, de préférence sur une partie du corps qui n'est pas le visage. L'invention sera illustrée plus en détail par les exemples suivants.

**Exemple 1 : Evaluation de la fidélité olfactive et de la rémanence d'une composition parfumante selon l'invention**

On a préparé la composition parfumante selon l'invention suivante.

[Tableau 1]

|  | Ingrédients | % en masse |
|---|---|---|
| Phase aqueuse | Eau | 86.7 |
|  | Hydroxyacétophénone | 0.5 |
|  | Chlrorphénésine | 0.3 |
| Phase grasse | Alcanes en C9-12 (Cetiol Ultimate® de BASF) | 2.5 |
|  | Concentré de parfum | 10 |

On a pesé et mélangé les matières premières de la phase aqueuse et de la phase grasse respectivement, avant de verser la phase grasse dans la phase aqueuse.

La composition parfumante est conditionnée dans un flacon équipé d'un dispositif de pulvérisation. La composition se présente au repos sous la forme d'un biphase, dont les deux phases transparentes non-miscibles sont visuellement distinctes.

Après agitation manuelle pour homogénéiser la composition de la composition, celle-ci est conditionnée dans un flacon comprenant un dispositif de pulvérisation.

La fidélité olfactive et l'intensité (rémanence) de la composition selon l'invention sont évaluées par un panel de quatre experts dotés d'une expérience de caractérisations objectives et descriptives des qualités sensorielles de compositions cosmétiques parfumées ou parfumantes.

On utilise comme Référence un parfum du commerce de type « floral » et de rémanence moyenne, qui contient 79% d'éthanol (MISS DIOR BLOOMING BOUQUET@), et un concentré de parfum floral. L'exemple de l'invention est préparé avec le même concentré que celui de la Référence

Pour chaque produit, l'intensité et la fidélité sont évaluées immédiatement après application sur la peau (T0), puis après 6 heures (T1) et 8 heures (T2). Pour chaque critère, l'expert donne une note sur une échelle de 0 à 9 (note optimale).

Le protocole est le suivant :

- on applique une seule pulvérisation de la composition de l'invention, à environ 5 cm du bras, sur la peau d'un avant-bras de l'expert, et on réalise la même application avec la Référence sur l'autre avant-bras,
- après évaluation à T0, on protège des frottements les deux zones de peau parfumées
- à T1 puis à T2, on évalue à nouveau ces mêmes critères.

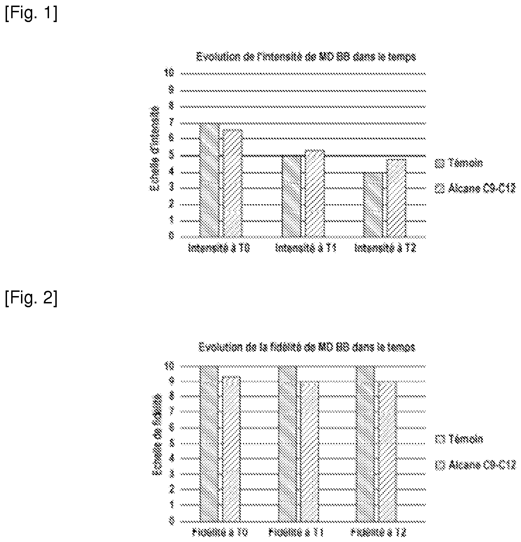

Les résultats ont été reportés aux Figures 1 et 2.

L'essai avec les alcanes en C9-C12 ressort plus rémanent que le témoin après 6h et 8h. Il est moins fusant et, moins frais que le témoin à T0. La fidélité de l'essai avec les alcanes en C9-C12 au témoin, est constante dans le temps.

### Exemple 2

On a préparé la composition parfumante selon l'invention suivante.

[Tableau 2]

| | Ingrédients | % en masse |
|---|---|---|
| Phase aqueuse | Eau | 87,1 |
| | Acide citrique | 0,1 |
| | Potassium sorbate | 0,2 |
| | Sodium benzoate | 0,1 |
| Phase grasse | Polyisobutène hydrogéné (POLYSYNLANE® 4) | 2,5 |
| | Concentré de parfum | 10 |

Le concentré de parfum utilisé dans cet exemple est le même que celui utilisé pour préparer le produit commercial MISS DIOR BLOOMING BOUQUET@.

On a pesé et mélangé les matières premières de la phase aqueuse et de la phase grasse respectivement, avant de verser la phase grasse dans la phase aqueuse.

La composition parfumante est conditionnée dans un flacon équipé d'un dispositif de pulvérisation. La composition se présente au repos sous la forme d'un biphase, dont les deux phases transparentes non-miscibles sont visuellement distinctes.

Après agitation manuelle pour homogénéiser la composition de la composition, celle-ci est conditionnée dans un flacon comprenant un dispositif de pulvérisation.

La composition est ainsi pulvérisée sur la peau ou les vêtements sans que l'identité olfactive ne soit altérée par la présence d'une huile alcane.

### Exemples 3 et 4

On a préparé les quatre compositions suivantes : deux formules conformes à l'art antérieur décrit dans la demande FR 2 952 531, et deux compositions selon l'invention.

[Tableau 3]

| | Formule 1 comparative | Formule 2 comparative | Exemple 3 | Exemple 4 |
|---|---|---|---|---|
| Copolymère réticulé d'AMPS et ester d'acide (méth)acrylique et d'alcool gras (Aristoflex® HMS) | 0,8 | 0,8 | 0 | 0 |
| Parfum | 2 | 2 | 4 | 8 |
| Conservateur | 0,2 | 0,2 | 0,2 | 0,2 |
| N-dodécane | 10 | | | |
| C9-12 ALKANE | | 10 | 10 | 10 |
| Eau | Qsp 100 | Qsp 100 | Qsp 100 | Qsp 100 |

Les formules comparatives 1 et 2 ont une texture épaisse et sont opaques de couleur blanche, tandis que les compositions des exemples 3 et 4 sont pulvérisables et transparentes.

### Revendications

1. Composition cosmétique parfumante liquide comprenant a) au moins 50% en masse d'eau, b) moins de 10% en masse d'éthanol, c) au moins 3% en masse d'un concentré de parfum , d) au moins une huile choisie parmi les alcanes, et e) moins de 0,1% en masse d'un agent émulsionnant, les pourcentages étant exprimés par rapport à la masse de la composition.

2. Composition selon la revendication 1, **caractérisée en ce que** l'huile alcane représente de 1% à 10%, de préférence de 2% à 7% en masse, encore mieux de 2,5% à 5% en masse de la masse de la composition.

3. Composition selon l'une des revendications précédentes, **caractérisée en ce qu'**elle contient de 8% en masse à 12% en masse de concentré de parfum, de 1% en masse à 5% en masse d'huile alcane et de l'eau dans une quantité telle que la somme des concentrations en concentré de parfum, en huile et en eau est supérieure à 99% en masse, les pourcentages étant exprimés par rapport à la masse de la composition.

4. Composition selon l'une des revendications précédentes, **caractérisée en ce que** l'huile est choisie parmi les alcanes en C8-C19, les polyisobutènes hydrogénés, le squalane, l'hemisqualane et leurs mélanges.

5. Composition selon l'une des revendications précédentes, **caractérisée en ce que** le ratio massique entre l'huile alcane et le concentré de parfum va de 1/10 à 2/1.

6. Composition selon l'une des revendications précédentes, **caractérisée en ce que** l'huile est choisie parmi :

   - les alcanes ramifiés en C8-C16 comme les iso-alcanes en C8-C16, l'isododécane, l'isodécane, l'isohexadécane,
   - les alcanes linéaires ou ramifés ayant des chaînes hydrocarbonées en C9-C17, C10-C14, C15-C19, C9-C12, C12-C14, le n-dodécane (C12) , le n-tétradécane (C14),
   - le squalane, l'hemisqualane,
   - les hydrocarbures comme les polyisobutènes hydrogénés, en particulier un polyisobutène hydrogéné ayant une masse moléculaire en masse inférieure à 250 g/mol,
   - et leurs mélanges.

7. Composition selon l'une des revendications précédentes, **caractérisée en ce que** l'huile alcane est volatile.

8. Composition selon l'une des revendications précédentes, **caractérisée en ce qu'**elle comprend moins de 0,05% en masse d'un agent tensioactif, de préférence moins de 0,01% en masse par rapport à la masse de la composition.

9. Composition selon l'une des revendications précédentes, **caractérisée en ce qu'**elle comprend au moins 60% en masse d'eau, de préférence au moins 65% en masse, mieux au moins 70% en masse d'eau par rapport à la masse de la composition.

10. Composition selon l'une des revendications précédentes, **caractérisée en ce que** la teneur en éthanol est inférieure ou égale à 5% en masse, de préférence inférieure ou égale à 2% en masse.

11. Composition selon l'une des revendications précédentes, **caractérisée en ce qu'**elle est exempte d'éthanol.

12. Composition selon l'une des revendications précédentes, **caractérisée en ce qu'**elle présente deux phases visuellement distinctes au repos.

13. Flacon comprenant un moyen d'application et un moyen de conditionnement, ledit moyen de conditionnement contenant la composition parfumante selon l'une des revendications précédentes.

14. Procédé cosmétique comprenant une étape d'application sur les matières kératiniques ou sur les vêtements d'une personne, d'une composition cosmétique selon l'une des revendications 1 à 12.

**Patentansprüche**

1. Flüssige Duftstoffzusammensetzung, umfassend:

   a) mindestens 50 Masse-% Wasser,
   b) mindestens 10 Masse-% Ethanol,
   c) mindestens 3 Masse-% eines Duftstoffkonzentrats,

d) mindestens ein Öl, das ausgewählt ist aus Alkanen, und

e) mindestens 0,1 Masse-% eines Emulgators,

wobei die Prozentsätze auf die Masse der Zusammensetzung bezogen sind.

2.	Zusammensetzung nach Anspruch 1, **dadurch gekennzeichnet, dass** das Alkanöl zwischen 1 und 10 Masse-%, vorzugsweise zwischen 2 und 7 Masse-%, noch besser zwischen 2,5 und 5 Masse-%, der Masse der Zusammensetzung ausmacht.

3.	Zusammensetzung nach einem der vorhergehenden Ansprüche, **dadurch gekennzeichnet, dass** sie zwischen 8 Masse-% und 12 Masse-% Duftstoffkonzentrat, zwischen 1 Masse-% und 5 Masse-% Alkanöl und Wasser in einer Menge enthält, die derart ist, dass die Summe der Konzentrationen an Duftstoffkonzentrat, an Öl und an Wasser größer als 99 Masse-% ist, wobei die Prozentsätze auf die Masse der Zusammensetzung bezogen sind.

4.	Zusammensetzung nach einem der vorhergehenden Ansprüche, **dadurch gekennzeichnet, dass** das Öl ausgewählt ist aus C8-C19-Alkanen, hydrierten Polyisobutenen, Squalan, Hemisqualan und ihren Gemischen.

5.	Zusammensetzung nach einem der vorhergehenden Ansprüche, **dadurch gekennzeichnet, dass** das Massenverhältnis zwischen dem Alkanöl und dem Duftstoffkonzentrat zwischen 1 : 10 und 2 : 1 beträgt.

6.	Zusammensetzung nach einem der vorhergehenden Ansprüche, **dadurch gekennzeichnet, dass** das Öl ausgewählt ist aus:

- verzweigten C8-C16-Alkanen, wie C8-C16-Isoalkanen, Isododecan, Isodecan, Isohexadecan,
- geradlinigen oder verzweigten Alkanen, die C9-C17-, C10-C14-, C15-C19-, C9-C12-, C12-C14-Kohlenwasserstoffketten aufweisen, n-Dodecan (C12), n-Tetradecan (C14),
- Squalan, Hemisqualan,
- Kohlenwasserstoffen, wie hydrierten Polyisobutenen, insbesondere einem hydrierten Polyisobuten mit einer Molekülmasse mit einer Masse von kleiner als 250 g/mol,
- und ihren Gemischen.

7.	Zusammensetzung nach einem der vorhergehenden Ansprüche, **dadurch gekennzeichnet, dass** das Alkanöl flüchtig ist.

8.	Zusammensetzung nach einem der vorhergehenden Ansprüche, **dadurch gekennzeichnet, dass** sie, bezogen auf die Masse der Zusammensetzung, weniger als 0,05 Masse-%, vorzugsweise weniger als 0,01 Masse-%, eines Tensids umfasst.

9.	Zusammensetzung nach einem der vorhergehenden Ansprüche, **dadurch gekennzeichnet, dass** sie, bezogen auf die Masse der Zusammensetzung, mindestens 60 Masse-% Wasser, vorzugsweise mindestens 65 Masse-%, besser mindestens 70 Masse-% Wasser, umfasst.

10.	Zusammensetzung nach einem der vorhergehenden Ansprüche, **dadurch gekennzeichnet, dass** der Gehalt an Ethanol kleiner oder gleich 5 Masse-%, vorzugsweise kleiner oder gleich 2 Masse-%, ist.

11.	Zusammensetzung nach einem der vorhergehenden Ansprüche, **dadurch gekennzeichnet, dass** sie frei von Ethanol ist.

12.	Zusammensetzung nach einem der vorhergehenden Ansprüche, **dadurch gekennzeichnet, dass** sie im Ruhezustand zwei visuell getrennte Phasen aufweist.

13.	Flakon, der ein Aufbringungsmittel und ein Verpackungsmittel umfasst, wobei das Verpackungsmittel die Duftstoffzusammensetzung nach einem der vorhergehenden Ansprüche enthält.

14.	Kosmetisches Verfahren, das einen Schritt des Aufbringens einer kosmetischen Zusammensetzung nach einem der Ansprüche 1 bis 12 auf das Keratinmaterial oder auf die Kleidung einer Person umfasst.

**EP 4 142 678 B1**

**Claims**

1. A liquid perfuming cosmetic composition comprising a) at least 50% by mass of water, b) less than 10% by mass of ethanol, c) at least 3% by mass of a perfume concentrate and d) at least one oil chosen from alkanes, and e) less than 0.1% by mass of an emulsifying agent, the percentages being expressed relative to the mass of the composition.

2. The composition according to claim 1, **characterized in that** the alkane oil represents from 1% to 10%, preferably from 2% to 7% by mass, better from 2.5% to 5% by mass of the composition.

3. The composition according to one of the preceding claims, **characterized in that** it contains from 8% by mass to 12% by mass of perfume concentrate, from 1% by mass to 5% by mass of alkane oil, and water in an amount such that the sum of the concentrations of perfume concentrate, oil and water is greater than 99% by mass, the percentages being expressed relative to the mass of the composition.

4. The composition according to one of the preceding claims, **characterized in that** the oil is chosen from C8-C19 alkanes, hydrogenated polyisobutenes, squalane, hemisqualane and mixtures thereof.

5. The composition according to one of the preceding claims, **characterized in that** the mass ratio between the alkane oil and the perfume concentrate ranges from 1:10 to 2:1.

6. The composition according to one of the preceding claims, **characterized in that** the oil is chosen from:

   - branched C8-C16 alkanes such as C8-C16 iso-alkanes, isododecane, isodecane, isohexadecane, linear or branched alkanes having hydrocarbon chains in C9-C17, C10-C14, C15-C19, C9-C12, C12-C14, n-dodecane (C12), n-tetradecane (C14),
   - squalane, hemisqualane,
   - hydrocarbons such as hydrogenated polyisobutene having a molecular weight of less than 250 g/mol,
   - and mixtures thereof.

7. The composition according to one of the preceding claims, **characterized in that** the alkane oil is volatile.

8. The composition according to one of the preceding claims, **characterized in that** it comprises less than 0.05% by mass of a surfactant, more preferably less than 0.01% by mass of the mass of the composition.

9. The composition according to one of the preceding claims, **characterized in that** it comprises at least 60% by mass of water, preferably at least 65% by mass, better at least 70% by mass of water relative to the mass of the composition.

10. The composition according to one of the preceding claims, **characterized in that** the ethanol content is less than or equal to 5% by mass, preferably less than or equal to 2% by mass.

11. The composition according to one of the preceding claims, **characterized in that** it is free of ethanol.

12. The composition according to one of the preceding claims, **characterized in that** it has two visually distinct phases on standing.

13. Vial provided with an application means and a packaging means, said packaging means containing the perfuming composition according to one of the preceding claims.

14. Cosmetic process comprising a step of applying to the keratin materials or clothing of an individual a cosmetic composition according to one of claims 1 to 12.

[Fig. 1]

Evolution de l'intensité de MD BB dans le temps

[Fig. 2]

Evolution de la fidélité de MD BB dans le temps

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2952531